# EUROPEAN PATENT APPLICATION

(11) **EP 1 867 276 A1**
(43) Date of publication of application: **19.12.2007**
(21) Application number: 06729899.2
(22) Date of filing: 24.03.2006
(51) Int. Cl.: A61B 5/022, A61B 5/0245

(54) **BLOOD PRESSURE MEASURING DEVICE AND BLOOD PRESSURE MEASURING METHOD**

(30) Priority: 08.04.2005 JP 2005112560
(71) Applicant: TERUMO KABUSHIKI KAISHA, Tokyo 151-0072 (JP); NIPPON TELEGRAPH AND TELEPHONE CORPORATION, Tokyo 100-8116 (JP)
(72) Inventor: HABU, Yoshiyuki c/o TERUMO KABUSHIKI KAISHA, Kanagawa 2590151 (JP); HAGI, Kouji c/o TERUMO KABUSHIKI KAISHA, Kanagawa 2590151 (JP); OZAWA, Hitoshi c/o TERUMO KABUSHIKI KAISHA, Shizuoka 4180015 (JP); HAYASHIDA, Shoichi NIPPON TEL. &TELEPHONE Corp., Tokyo 1008116 (JP); SHIMADA, Junichi NIPPON TELEGRAPH & TELEPHONE Corp, Chiyoda-ku Tokyo 1008116 (JP); KOIZUMI, Hiroshi NIPPON Telegraph & Tel. Corp., Chiyoda-ku Tokyo 1008116 (JP)
(74) Representative: Paget, Hugh Charles Edward
(86) International application number: PCT/JP2006/305951
(87) International publication number: WO 2006/109519

(57) **Abstract**

This invention provides a blood pressure measuring apparatus capable of deriving an appropriate pressure value even in an environment in which the environmental temperature constantly changes.

A blood pressure measuring apparatus has a cuff to be attached to the external ear and its periphery, a signal acquiring unit which acquires a pulse wave signal and/or Korotkoff sounds from a pressed portion and its periphery pressed by the cuff, a pressure sensor which outputs a predetermined signal level corresponding to a pressure value in the cuff, a temperature measuring unit which measures a temperature near the pressure sensor, a blood pressure value deriving unit which derives a blood pressure value on the basis of a pressure value derived from the signal level, and the pulse wave signal and/or the Korotkoff sounds, and an adjusting unit which performs adjustment which compensates for a characteristic change of the pressure sensor with respect to the pressure value, if the temperature measured by the temperature measuring unit changes by a predetermined value or more.

## Description

### TECHNICAL FIELD

The present invention relates to a blood pressure measuring technique and, more particularly, to a technique of adjusting the derivation of a cuff pressure value from an output signal from a pressure sensor.

### BACKGROUND ART

The conventional electronic sphygmomanometer uses a pressure sensor that outputs a pressure value change as a voltage value change, in order to measure the internal pressure of a cuff (to control the cuff pressure or detect the pressure pulse wave). Generally, the pressure sensor changes the pressure value-voltage value output characteristic when its own temperature changes owing to the external environment. As described in patent reference 1, therefore, the pressure value is derived from the voltage value using an adjusting method that adds a predetermined compensation amount whenever a necessary time has passed.
Patent reference 1: Japanese Patent Publication No. 58-34159

### DISCLOSURE OF INVENTION

### PROBLEMS THAT THE INVENTION IS TO SOLVE

It is assumed that a blood pressure measuring apparatus that uses the external ear and its periphery as measurement portions is used in continuous long-time measurements. In this case, the measuring apparatus moves indoors and outdoors day and night together with a person to be measured. Accordingly, the environmental temperature of the pressure sensor presumably changes largely owing to the change in the external temperature or the change in body temperature. This makes it important to compensate for the pressure value by adjusting the temperature characteristic of the pressure sensor.

Unfortunately, when the blood pressure measuring apparatus is used in an environment in which the temperature constantly changes, the conventional adjusting technique designed based on the assumption that the blood pressure measuring apparatus is used indoors cannot retain the same level of accuracy. Also, since the adjusting operation generally requires a long time, the psychological and physical burdens on a person to be measured increase if the frequency of the adjusting operation increases.

The present invention has been made in consideration of the above problems, and provides a technique capable of deriving an appropriate pressure value by compensating for, by adjustment, the characteristic change, caused by the temperature, of a pressure sensor for deriving the internal pressure value of a cuff when measuring the blood pressure, even when used in an environment in which the temperature constantly changes. The present invention also provides a technique capable of decreasing the frequency of an adjusting operation, thereby reducing the psychological and physical burdens on a user when performing the adjusting operation.

### MEANS OF SOLVING THE PROBLEMS

A blood pressure measuring apparatus comprises a cuff to be attached to an external ear and its periphery, signal acquiring means for acquiring a pulse wave signal and/or Korotkoff sounds from a pressed portion and its periphery pressed by the cuff, a pressure sensor which outputs a predetermined signal level corresponding to a pressure value in the cuff, blood pressure value deriving means for deriving a blood pressure value on the basis of a pressure value derived from the signal level, and the pulse wave signal and/or the Korotkoff sounds, and adjusting means for performing adjustment which compensates for a characteristic change of the pressure sensor with respect to the pressure value, if the signal level changes by a predetermined value or more when the cuff is not pressurized.

A blood pressure measuring apparatus comprises a cuff to be attached to an external ear and its periphery, signal acquiring means for acquiring a pulse wave signal and/or Korotkoff sounds from a pressed portion and its periphery pressed by the cuff, a pressure sensor which outputs a predetermined signal level corresponding to a pressure value in the cuff, temperature measuring means for measuring a temperature near the pressure sensor, blood pressure value deriving means for deriving a blood pressure value on the basis of a pressure value derived from the signal level, and the pulse wave signal and/or the Korotkoff sounds, and adjusting means for performing adjustment which compensates for a characteristic change of the pressure sensor with respect to the pressure value, if the temperature measured by the temperature measuring means changes by a predetermined value or more.

A blood pressure measuring apparatus comprises a cuff to be attached to an external ear and its periphery, signal acquiring means for acquiring a pulse wave signal and/or Korotkoff sounds from a pressed portion and its periphery pressed by the cuff, a pressure sensor which outputs a predetermined signal level corresponding to a pressure value in the cuff, temperature measuring means for measuring a temperature near the pressure sensor, blood pressure value deriving means for deriving a blood pressure value on the basis of a pressure value derived from the signal level, and the pulse wave signal and/or the Korotkoff sounds, and adjusting means for performing adjustment which compensates for a characteristic change of the pressure sensor with respect to the pressure value, if the signal level changes by a predetermined value or more when the cuff is not pressurized, and if the temperature measured by the temperature measuring means changes by a predetermined value or more.

A blood pressure measuring method comprises a pressing step of pressing an external ear and its periphery by a cuff, a signal acquiring step of acquiring a pulse wave signal and/or Korotkoff sounds from a pressed portion and its periphery pressed in the pressing step, a signal output step of outputting, from a pressure sensor, a predetermined signal level corresponding to a pressure value in the cuff, a temperature measuring step of measuring a temperature near the pressure sensor, a blood pressure value deriving step of deriving a blood pressure value on the basis of a pressure value derived from the signal level, and the pulse wave signal and/or the Korotkoff sounds, and an adjusting step of performing adjustment which compensates for a characteristic change of the pressure sensor with respect to the pressure value, if the temperature measured in the temperature measuring step changes by a predetermined value or more.

### EFFECTS OF THE INVENTION

The present invention can provide a technique capable of deriving an appropriate pressure value by compensating for, by adjustment, the characteristic change, caused by the temperature, of a pressure sensor for deriving the internal pressure value of a cuff when measuring the blood pressure. The present invention can also provide a technique capable of decreasing the frequency of an adjusting operation, thereby reducing the psychological and physical burdens on a user when performing the adjusting operation.

Other features and advantages of the present invention will be apparent from the following explanation taken in conjunction with the accompanying drawings. Note that the same reference numerals denote similar arrangements or the same arrangements in the accompanying drawings.

### BRIEF DESCRIPTION OF DRAWINGS

The accompanying drawings are included in the specification, constitute part of the specification, illustrate embodiments of the present invention, and are used to explain the principle of the present invention together with the description.

Fig. 1 is a block diagram of a photoelectric volume pulse wave sphygmomanometer of the first embodiment;
Fig. 2 is a perspective view showing the outer appearance of the photoelectric volume pulse wave sphygmomanometer of the first embodiment;
Fig. 3 is a view showing an example of attachment of a cuff to the external ear and its periphery;
Fig. 4A is a flowchart showing the overall operation of blood pressure measurement of the first embodiment;
Fig. 4B is a flowchart showing the overall operation of blood pressure measurement of the first embodiment;
Fig. 5 is a flowchart showing the pressure value adjusting operation of the first embodiment;
Fig. 6 is a graph showing examples of the temperature characteristics of a pressure sensor;
Fig. 7 is a graph showing the relationship between a pulse wave signal and a blood pressure value;
Fig. 8 is a block diagram of a pressure pulse wave sphygmomanometer of the second embodiment;
Fig. 9 is a flowchart showing the pressure value adjusting operation of the second embodiment; and
Fig. 10 is a flowchart showing the pressure value adjusting operation of the third embodiment.

### BEST MODE FOR CARRYING OUT THE INVENTION

Preferred embodiments of the present invention will be explained in detail below as examples with reference to the accompanying drawings. However, constituent elements described in these embodiments are merely examples, so the scope of the present invention is not limited to these constituent elements.

### (First Embodiment)

The first embodiment of an electronic sphygmomanometer according to the present invention will be explained below by taking a photoelectric volume pulse wave sphygmomanometer as an example.

### <Apparatus Arrangement>

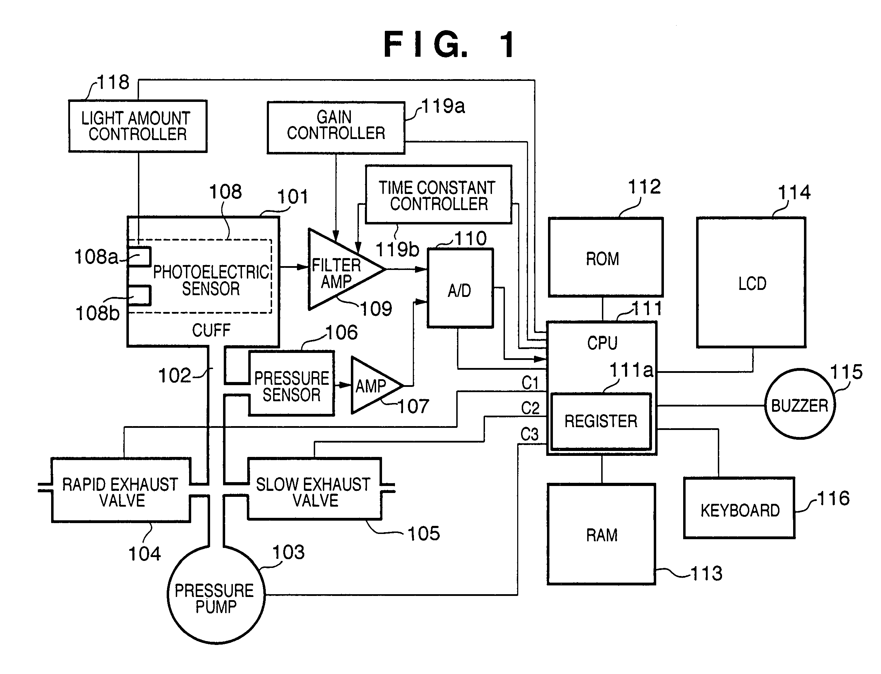

Fig. 1 is a block diagram showing the arrangement of the photoelectric volume pulse wave sphygmomanometer of the embodiment. Referring to Fig. 1, a cuff 101 is fixed to a blood pressure measurement portion. An air tube 102 forms a channel of air to the cuff 101. A pressure pump 103 supplies pressurized air into the cuff 101. A rapid exhaust valve 104 rapidly reduces the internal pressure of the cuff 101. A slow exhaust valve 105 reduces the internal pressure of the cuff 101 at a constant rate (e.g., 2 to 3 mmHg/sec). A pressure sensor 106 changes an electrical parameter in accordance with the internal pressure of the cuff 101. A pressure detection amplifier (AMP) 107 detects the electrical parameter of the pressure sensor 106, converts the parameter into an electrical signal, and amplifies the signal, thereby outputting an analog cuff pressure signal P (not shown).

A pulse wave sensor 108 installed in the cuff 101 includes an LED 108a that irradiates a pulsing blood flow with light, and a phototransistor 108b that detects the reflected light from the blood flow. A pulse wave detection amplifier (AMP) 109 amplifies the output signal from the phototransistor 108b, and outputs an analog pulse wave signal M (not shown). The LED 108a is connected to a light amount controller 118 that automatically changes the light amount, and the pulse wave detection amplifier 109 is connected to a gain controller 119a that automatically changes the gain, and a time constant controller 119b that changes the time constant. An A/D converter (A/D) 110 converts the analog signals M and P (not shown) into digital data.

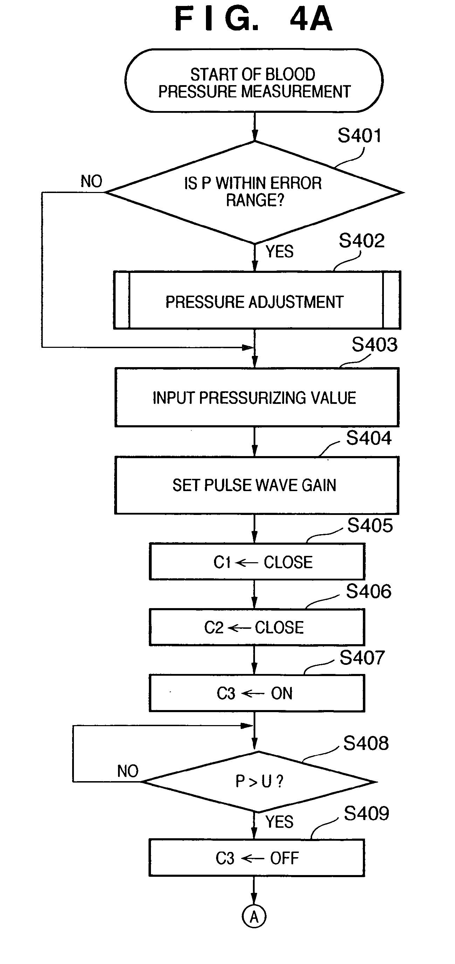

A controller (CPU) 111 performs main control of this photoelectric volume pulse wave sphygmomanometer. Details of this control will be described later with reference to flowcharts shown in Figs. 4A to 5. A ROM 112 stores various control programs executed by the CPU 111, and various parameters (e.g., a parameter for deriving a pressure value from the output signal from the pressure sensor). An example of the various control programs is a program that performs the control shown in Figs. 4A to 5 executed by the CPU 111. A RAM 113 includes, e.g., a data memory that temporarily stores data, and an image memory for displaying images. A liquid crystal display (LCD) 114 displays the contents of the image memory. A keyboard 116 is operated by the user to, e.g., input a measurement start command or set an adjusted pressure value. A buzzer 115 notifies the user that, e.g., the apparatus has sensed pressing of a key on the keyboard 116, or the measurement is complete. Note that although an adjusted pressure register 111a is allocated in the CPU 111 in this embodiment, an adjusted pressure storage unit may also be allocated in the RAM 113.

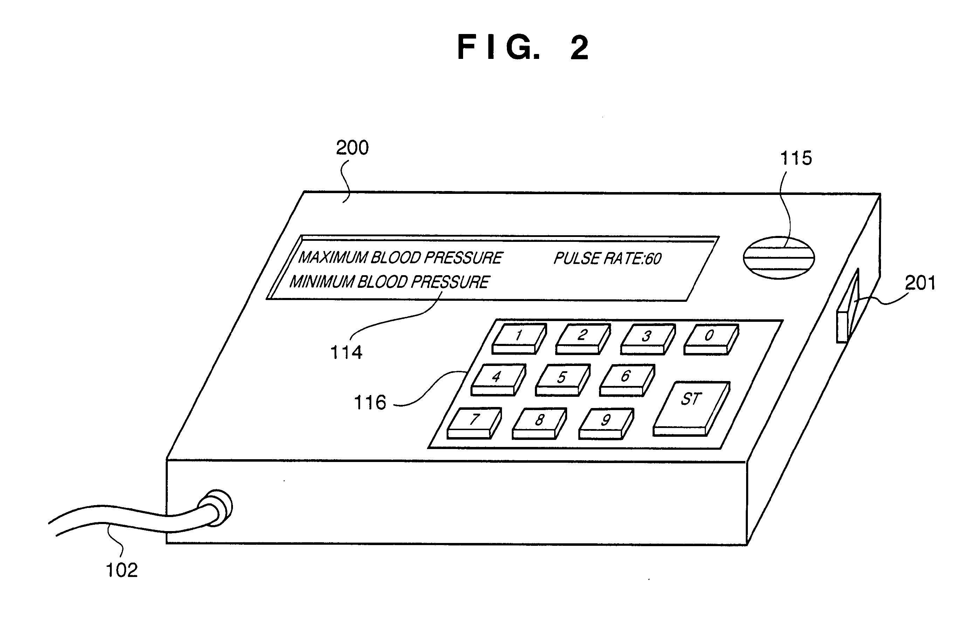

Fig. 2 is a perspective view showing the outer appearance of the photoelectric volume pulse wave sphygmomanometer of the embodiment. A sphygmomanometer main body 200 includes components except for the cuff 101 and pulse wave sensor 108 shown in Fig. 1. The air tube 102 includes signal lines (not shown), and connects to the cuff 101 and pulse wave sensor 108 (neither is shown). A dot matrix type display panel is used as the LCD 114, so the LCD 114 can display various kinds of information (e.g., characters, figures, and signal waveforms). Reference numeral 201 denotes a power switch. The keyboard 116 has a measurement start switch (ST) and a ten-key pad for inputting the cuff pressure value and the like.

### <Method of Attachment to Measurement Portion>

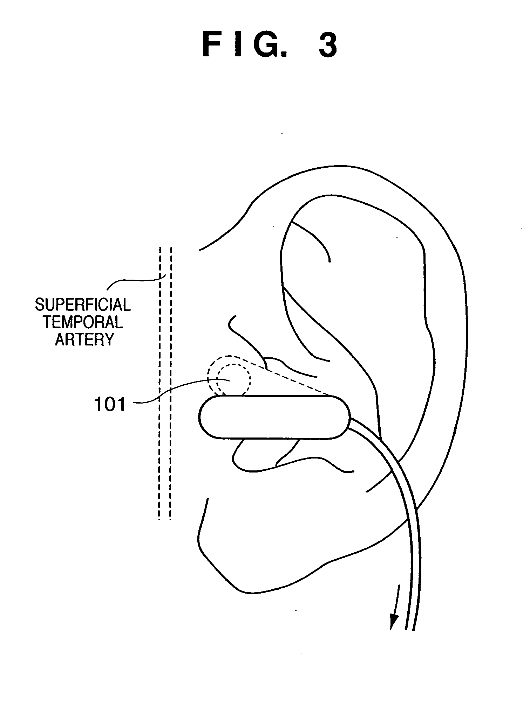

Since the external ear, particularly, the tragus and its periphery are measurement portions, the measuring unit including the cuff is constructed to clamp and press the tragus from the two sides as shown in Fig. 3.

### <Blood Pressure Measuring Operation of Apparatus>

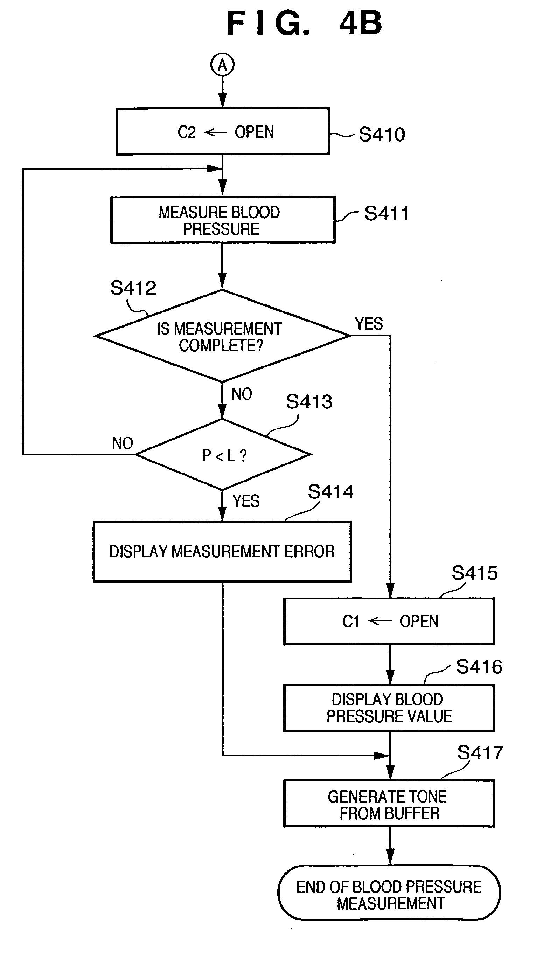

The operation of the photoelectric volume pulse wave sphygmomanometer according to this embodiment will be explained below. Figs. 4A and 4B are flowcharts showing the blood pressure measurement procedure in the photoelectric volume pulse wave sphygmomanometer of the first embodiment.

When the apparatus is switched on by the power switch 201, the CPU 111 first reads out a self-diagnosing program and initial parameters stored in the ROM 112, initializes the apparatus by performing a self-diagnosing process, and enters a waiting state. When the user presses the measurement start switch ST after that, the blood pressure measurement process starts.

In step S401, the cuff pressure value P is derived from the pressure sensor, and whether the derived value P falls within a predetermine error range from 0 mmHg is determined. If the value P falls outside the error range, the process advances to step S402. If the value P falls within the error range, the process advances to step S403.

In step S402, pressure value derivation adjustment is performed because the value P derived in step S401 falls outside the error range, and the process advances to step S403. The operation of this pressure value derivation adjustment will be explained in detail later.

In step S403, the user sets a pressurization value U (e.g., a value larger than a maximum blood pressure value of 120 to 280 mmHg) of the cuff by using the keyboard 116. In step S404, the gain (the light amount and gain) of a pulse wave signal is set.

After the pressurization value and gain are set, the rapid exhaust valve 104 and slow exhaust valve 105 are respectively closed in steps S405 and S406. In step S407, driving of the pressure pump 103 is started. In step S408, the cuff pressure is increased until P > U. If P > U, the pressure pump 103 is stopped in step S409, and the slow exhaust valve 105 is opened in step S410.

The cuff pressure starts reducing at a constant rate (e.g., 2 to 3 mmHg/sec), and a blood pressure measurement process starts. In step S411, the individual functional blocks perform data processing, and measure the maximum blood pressure and minimum blood pressure. In step S412, whether the minimum blood pressure value is detected upon depressurization is determined. If no value is detected, the measurement continues. In step S413, whether the cuff pressure is lower than a predetermined value L (e.g., 40 mmHg) is determined. If P ≥ L, the cuff pressure still falls within the normal measurement range, so the process returns to step S411. If P < L, the cuff pressure is lower than the normal measurement range, so the LCD 114 displays "measurement error" in step S414. If necessary, the LCD 114 additionally displays detailed information such as "signal abnormality upon depressurization".

If it is determined in step S412 that the measurement is complete, this means that the measurement process is completed within the normal measurement range. Accordingly, the remaining air in cuff 101 is rapidly exhausted in step S415, the LCD 114 displays the measured maximum blood pressure value and minimum blood pressure value in step S416, and a tone signal is supplied to the buzzer 115 in step S417. Preferably, different tone signals are supplied for normal termination and abnormal termination, the measurement is terminated, and the start of the next measurement is waited for.

### <Details of Operation of Pressure Value Adjustment>

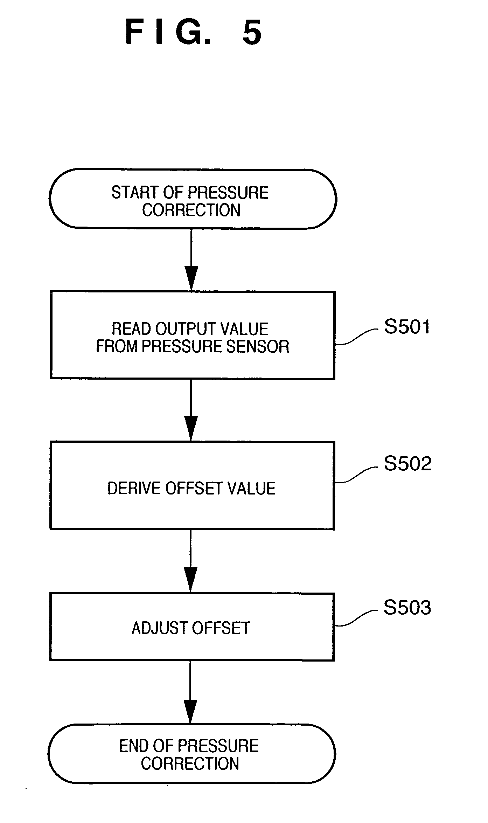

Fig. 5 is a flowchart for explaining details of the operation in step S402, which is the operation of correcting the pressure sensor.

Pressure value adjustment is executed if a pressure value equal to or larger than a predetermined error is derived after the apparatus is initialized although the pressure value should be 0 mmHg because the internal air of the cuff originally communicates with the atmosphere.

In step S501, the signal output value of the pressure sensor is read.

In step S502, an offset value by which a pressure value corresponding to the signal output value read in step S501 is 0 mmHg is derived. That is, the difference between the read signal output voltage value and the voltage value by which the pressure value is 0 mmHg as an initial parameter is calculated.

In step S503, the parameter stored in the ROM 112 is replaced with a new parameter on the basis of the offset value derived in step S502.

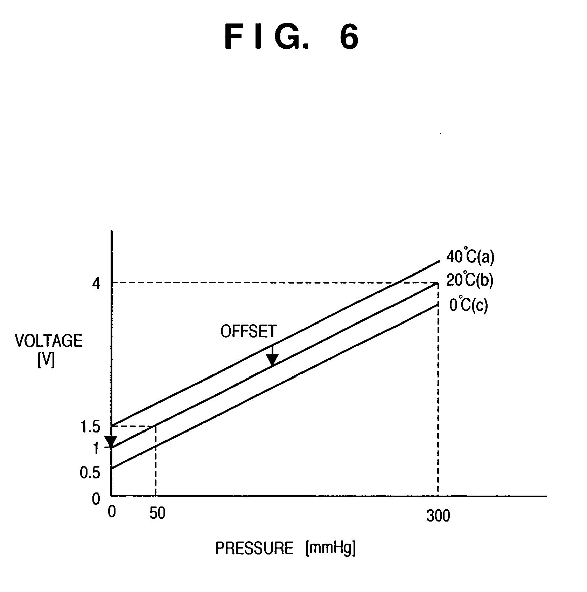

An operation related to the above pressure adjusting operation will be explained below by taking, as an example, the case that a pressure sensor having temperature characteristics as shown in Fig. 6 is used in a place where the external temperature is 40°C.

A pressure value derivation parameter corresponding to a temperature characteristic as indicated by (b) is stored as an initial parameter in the ROM 112. However, when measurement is performed in a place where the external temperature is 40°C, the temperature of the pressure sensor itself rises to about 40°C, so the output signal of the pressure sensor immediately after the blood pressure measuring apparatus is initialized is 1.5 V. When this result is converted into a pressure value by using the parameter (b), a pressure value of 50 mmHg is obtained. If blood pressure measurement is performed by using the parameter (b), a pressure higher by about 50 mmHg is obtained as a result.

While the internal air of the cuff originally communicates with the atmosphere after the apparatus is initialized, therefore, the output value (in this case, 1.5 V) of the signal from the pressure sensor is read in step S501.

In step S502, the offset value (0.5 V) between the signal output value (1.0 V) corresponding to 0 mmHg obtained by the initial parameter (b) and the value (1.5 V) read in step S501 is derived. In step S503, a parameter changed by the offset value derived in step S502 is reset for the initial parameter presently being loaded.

Consequently, a parameter corresponding to a temperature characteristic (a) shown in Fig. 6 is set when the temperature is 40°C. This makes it possible to derive 0 mmHg as a correct pressure value even for the signal output value (1.5 V) of the pressure sensor in the state in which the internal air of the cuff communicates with the atmosphere.

Note that the correcting operation that derives a corrected pressure value by changing the derivation parameter in accordance with the sensor output value corresponding to the temperature change has been explained above. However, it is also possible to correct the output value itself from the pressure sensor by performing feedback control on the pressure sensor.

### <Details of Operation of Blood Pressure Measurement>

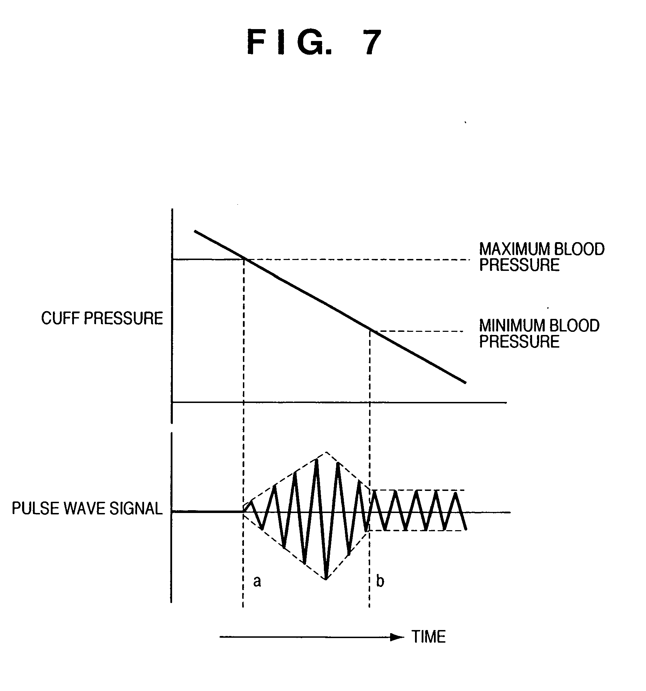

Fig. 7 shows a graph (schematic view) of the cuff pressure and pulse wave signal from the start to the end of blood pressure measurement when the measurement is normally done. The maximum blood pressure and minimum blood pressure are generally obtained as follows from the graph shown in Fig. 7. That is, the cuff pressure at the point (a) at which the pulse wave signal appears is the maximum blood pressure, and the cuff pressure at the point (b) at which the pulse wave signal stops changing its magnitude is the minimum blood pressure.

This embodiment has explained the example in which the reflected light from the blood in the blood vessel is detected, but it is also possible to detect transmitted light instead. In this case, 108a and 108b are arranged on the two sides of the measuring unit so as to sandwich it.

As explained above, the photoelectric volume pulse wave sphygmomanometer of this embodiment can derive an appropriate pressure value by compensating for the characteristic change, caused by the temperature, of the pressure sensor used to derive the internal pressure value of the cuff when measuring the blood pressure. It is also possible to provide a blood pressure measuring apparatus capable of decreasing the frequency of the adjusting operation, thereby reducing the psychological and physical burdens on a user when performing the adjusting operation.

### (Second Embodiment)

The second embodiment of the electronic sphygmomanometer according to the present invention will be explained below by taking a pressure pulse wave sphygmomanometer as an example. Note that this embodiment differs from the first embodiment in that temperature information obtained by a temperature sensor is mainly used. A method of attaching a cuff to a measurement portion and an operation of calculating the blood pressure are almost the same as in the first embodiment, so a repetitive explanation will be omitted.

### <Apparatus Arrangement>

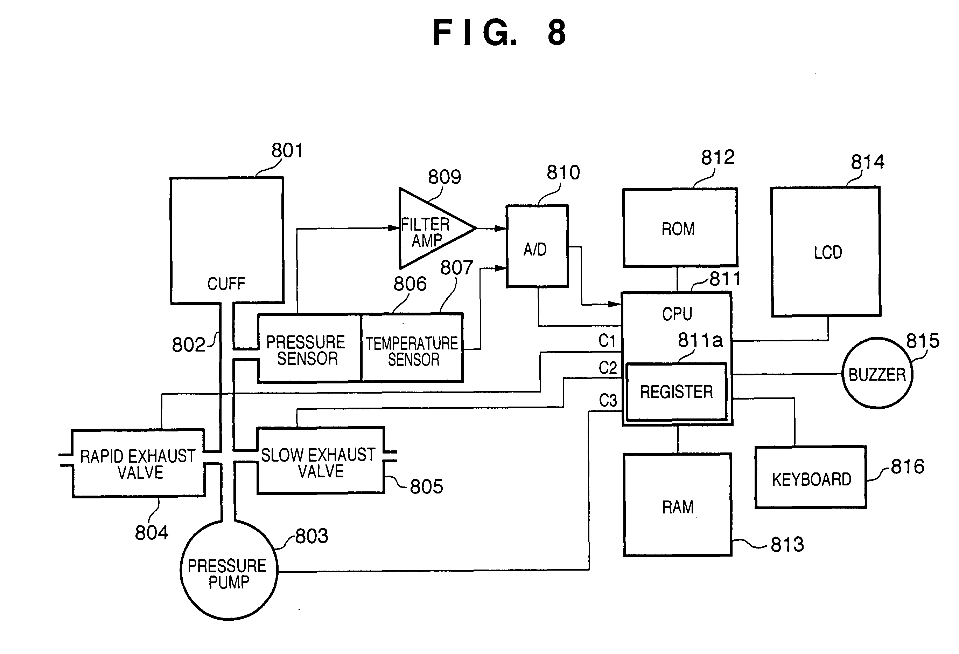

Fig. 8 is a block diagram showing the arrangement of the pressure pulse wave sphygmomanometer of the second embodiment. The differences from Fig. 1 are that the apparatus does not include a photoelectric sensor and its relevant portions, and has a temperature sensor 807 for acquiring the temperature of a pressure sensor 806.

Also, a plurality of parameters for deriving a pressure value from the output signal from the pressure sensor 806 are stored in a ROM 812. When setting a parameter, a CPU 811 stores the corresponding temperature value of the pressure sensor 806 in a temperature storage area of a RAM 813.

The rest of the arrangement is almost the same as the first embodiment, so an explanation will not be repeated.

### <Details of Operation of Pressure Value Adjustment>

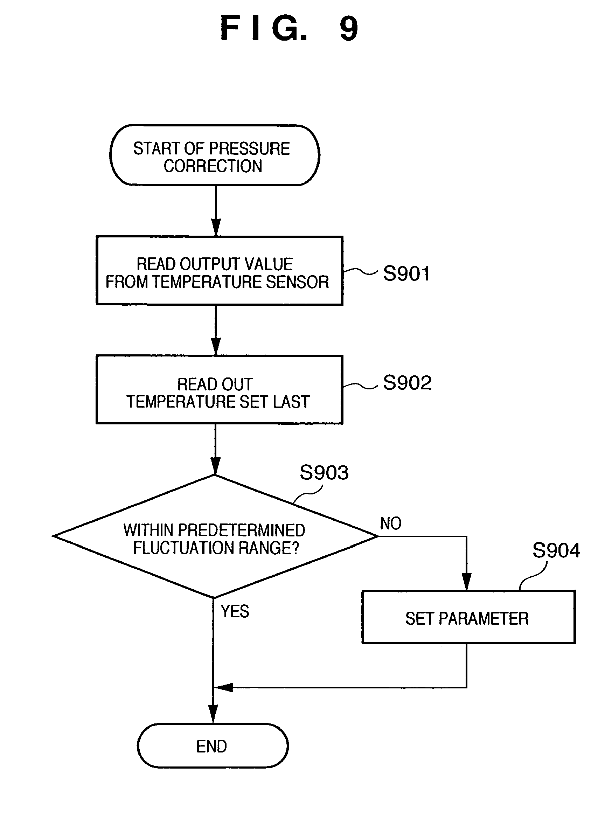

Fig. 9 is a flowchart for explaining details of the operation in step S402 in the pressure value adjusting operation. Pressure value adjustment is executed if a pressure value equal to or larger than a predetermined error is derived after the apparatus is initialized although the pressure value should be 0 mmHg because the internal air of the cuff originally communicates with the atmosphere, and includes the following steps.

In step S901, the temperature of the pressure sensor 806 is read by using the temperature sensor 807.

In step S902, the temperature value stored in the temperature storage area of the RAM is read out. That is, in the first measurement after the power supply is turned on, the temperature value set upon initialization is read out. In the second or subsequent measurement, the temperature value set in the last pressure adjustment is read out.

In step S903, whether the difference between the temperature value read in step S901 and the temperature value read out in step S902 is equal to or smaller than a predetermined value is determined. If the difference is equal to or smaller than the predetermined value, it is regarded that the characteristics of the pressure sensor remain unchanged, and the existing parameter is directly used without any new adjustment. If the difference is larger than the predetermined value, the process advances to step S904. Note that if the measurement accuracy is important, the predetermined value is desirably determined to be equal to or smaller than the measurement error (standard deviation) of the temperature sensor. If it is important to shorten the measurement time, a value larger than the measurement error of the temperature sensor may also be used.

In step S904, that parameter of the pressure sensor which corresponds to the temperature value read in step S901 is read out from the ROM 812, and overwritten on the existing parameter stored in the RAM 813.

The operations in the above steps will be explained below by taking, as an example, the case that a pressure sensor having temperature characteristics as indicated by (a) to (c) in Fig. 6 is used in a place where the external temperature is 40°C as in the first embodiment.

Pressure value derivation parameters corresponding to (a) to (c) in Fig. 6 are stored in the ROM 812 as parameters indicating the temperature characteristics of the pressure sensor.

When measurement is performed in a place where the external temperature is 40°C while the parameter corresponding to (b) is read out as the initial value, the temperature of the pressure sensor itself rises to almost 40°C. Consequently, the output signal from the pressure sensor is 1.5 V in accordance with (a) immediately after the blood pressure measuring apparatus is initialized, i.e., in the state in which the pressure sensor communicates with the atmosphere.

When this result is converted into a pressure value by using the parameter corresponding to the readout temperature characteristic (b), a pressure value of 50 mmHg is obtained. That is, if blood pressure measurement is performed by using the parameter corresponding to the temperature characteristic (b), a pressure higher by about 50 mmHg than the correct value is obtained as a result, so pressure value adjustment is necessary.

A practical operation example of the pressure value adjustment will be described below. While the internal air of the cuff originally communicates with the atmosphere after the apparatus is initialized, the temperature sensor reads the temperature (in this case, 40°C) of the pressure sensor in step S901.

In step S902, the temperature value (20°C) set and stored upon initialization is read out from the temperature value storage area of the RAM 813.

In step S903, whether the difference between the temperature value (40°C) read in step S901 and the temperature value (20°C) read out in step S902 is equal to or smaller than a predetermined value (e.g., 1°C) is determined. Since the difference is 15°C in this case, it is determined that the difference is larger than the predetermined value.

In step S904, the parameter corresponding to (a) of the pressure sensor, which corresponds to the temperature value (40°C) read in step S901, is read out from the ROM 812 and set.

Consequently, the parameter corresponding to (a) is set when the temperature is 40°C, so 0 mmHg as a correct pressure value can be derived even for the output value (1.5 V) of the signal from the pressure sensor in the state in which the internal air of the cuff communicates with the atmosphere.

Note that in Fig. 6, the temperature characteristic of the pressure sensor is explained by using a single parameter by assuming that the characteristic changes as a linear function, for the sake of descriptive simplicity. However, it is of course also possible to associate the temperature characteristic of the pressure sensor to a function of higher degree and express the characteristic by using a plurality of parameters.

Also, the start of blood pressure measurement is the trigger of temperature measurement (and the adjusting operation) in the above embodiment. However, it is also possible to continuously or periodically measure the temperature in the waiting state (i.e., the state in which a rapid exhaust valve 804 is open and the internal air of the cuff communicates with the atmosphere), and appropriately perform the adjusting operation if a predetermined temperature difference is produced. The result is the advantage that the measurement can be rapidly started.

### (Third Embodiment)

When no abnormality or the like occurs in the apparatus, it is possible to correct the pressure sensor characteristic and derive a highly accurate blood pressure value by using the pressure value change as explained in the first embodiment and the temperature value change as explained in the second embodiment.

On the other hand, if some abnormality occurs in the apparatus and a value that is supposed to be obtained cannot be obtained, not only the pressure sensor cannot be corrected, but also a value that is abnormal compared to that before correction may be obtained. Examples of the apparatus abnormality are a failure (output of an abnormal value) of the pressure sensor or temperature sensor, and a failure of an opening/closing valve. Therefore, it is important to sense any such abnormality of the apparatus and notify the user of it.

Accordingly, the third embodiment will explain an arrangement capable of compensating for the change in characteristic caused by the temperature change, and capable of sensing the apparatus abnormality, by monitoring both the pressure value change and temperature value change.

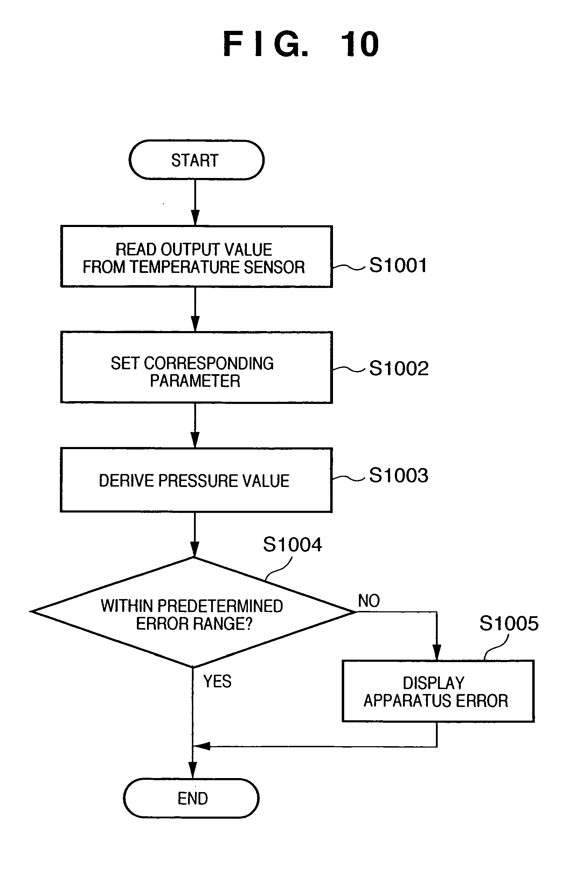

Fig. 10 is a flowchart of the pressure value adjusting operation of the third embodiment.

In step S1001, the temperature of a pressure sensor 806 is read by using a temperature sensor 807.

In step S1002, a pressure sensor parameter corresponding to the temperature value read in step S1001 is read out from a ROM 812 and stored in a RAM 813.

In step S1003, a pressure value is derived by using the parameter read out in step S1002.

In step S1004, whether the pressure value derived in step S1003 falls within a predetermined error range from 0 mmHg is determined. If the pressure value falls outside the predetermined error range although correction is performed in accordance with the temperature of the pressure sensor, it is concluded that some apparatus abnormality (the abnormality of the pressure sensor, temperature sensor, or opening/closing valve) has occurred. Therefore, an apparatus error is immediately displayed, and the measurement operation is terminated (interrupted).

If the pressure value derived in step S1003 falls within the predetermined error range from 0 mmHg, a normal blood pressure measurement operation starts.

As has been explained above, this embodiment makes it possible to properly correct pressure value derivation in an environment in which the temperature changes, and notify the user of a more appropriate blood pressure value.

Also, if an apparatus abnormality pertaining to pressure value derivation or temperature value derivation occurs, it is possible to sense the abnormality and notify the user of it.

The present invention is not limited to the above embodiments, and various changes and modifications can be made without departing from the spirit and scope of the invention. Therefore, to apprise the public of the scope of the present invention, the following claims are made.

## Claims

1. A blood pressure measuring apparatus **characterized by** comprising:
a cuff to be attached to an external ear and a periphery thereof;
signal acquiring means for acquiring a pulse wave signal and/or Korotkoff sounds from a pressed portion and a periphery thereof pressed by said cuff;
a pressure sensor which outputs a predetermined signal level corresponding to a pressure value in said cuff;
blood pressure value deriving means for deriving a blood pressure value on the basis of a pressure value derived from the signal level, and the pulse wave signal and/or the Korotkoff sounds; and
adjusting means for performing adjustment which compensates for a characteristic change of said pressure sensor with respect to the pressure value, if the signal level changes by not less than a predetermined value when said cuff is not pressurized.

2. A blood pressure measuring apparatus **characterized by** comprising:
a cuff to be attached to an external ear and a periphery thereof;
signal acquiring means for acquiring a pulse wave signal and/or Korotkoff sounds from a pressed portion and a periphery thereof pressed by said cuff;
a pressure sensor which outputs a predetermined signal level corresponding to a pressure value in said cuff;
temperature measuring means for measuring a temperature near said pressure sensor;
blood pressure value deriving means for deriving a blood pressure value on the basis of a pressure value derived from the signal level, and the pulse wave signal and/or the Korotkoff sounds; and
adjusting means for performing adjustment which compensates for a characteristic change of said pressure sensor with respect to the pressure value, if the temperature measured by said temperature measuring means changes by not less than a predetermined value.

3. A blood pressure measuring apparatus **characterized by** comprising:
a cuff to be attached to an external ear and a periphery thereof;
signal acquiring means for acquiring a pulse wave signal and/or Korotkoff sounds from a pressed portion and a periphery thereof pressed by said cuff;
a pressure sensor which outputs a predetermined signal level corresponding to a pressure value in said cuff;
temperature measuring means for measuring a temperature near said pressure sensor;
blood pressure value deriving means for deriving a blood pressure value on the basis of a pressure value derived from the signal level, and the pulse wave signal and/or the Korotkoff sounds; and
adjusting means for performing adjustment which compensates for a characteristic change of said pressure sensor with respect to the pressure value, if the signal level changes by not less than a predetermined value when said cuff is not pressurized, and if the temperature measured by said temperature measuring means changes by not less than a predetermined value.

4. A blood pressure measuring apparatus according to claim 1, **characterized in that** said adjusting means performs the adjustment on the basis of a pressure value of a signal level output from said pressure sensor when said cuff is not pressurized.

5. A blood pressure measuring apparatus according to claim 2, **characterized by** further comprising:
storage means for storing not less than one characteristic parameter corresponding to the temperature characteristic of said pressure sensor; and
selecting means for selecting a characteristic parameter corresponding to the temperature obtained by said temperature measuring means, from not less than one characteristic parameter stored in said storage means,
wherein said adjusting means adjusts said pressure value deriving means on the basis of the selected characteristic parameter.

6. A blood pressure measuring apparatus according to claim 1, **characterized in that** the pulse wave signal obtained by said signal acquiring means is a photoelectric pulse wave signal obtained by a photoelectric sensor.

7. A blood pressure measuring apparatus according to claim 1, **characterized in that** the external ear and the periphery thereof include one of a superficial temporal artery and a periphery of a branch thereof.

8. A blood pressure measuring method **characterized by** comprising:
a pressing step of pressing an external ear and a periphery thereof by a cuff;
a signal acquiring step of acquiring a pulse wave signal and/or Korotkoff sounds from a pressed portion and a periphery thereof pressed in the pressing step;
a signal output step of outputting, from a pressure sensor, a predetermined signal level corresponding to a pressure value in the cuff;
a temperature measuring step of measuring a temperature near the pressure sensor;
a blood pressure value deriving step of deriving a blood pressure value on the basis of a pressure value derived from the signal level, and the pulse wave signal and/or the Korotkoff sounds; and
an adjusting step of performing adjustment which compensates for a characteristic change of the pressure sensor with respect to the pressure value, if the temperature measured in the temperature measuring step changes by not less than a predetermined value.
